# EUROPÄISCHE PATENTSCHRIFT

(11) **EP 1 501 525 B1**
(45) Veröffentlichungstag und Bekanntmachung des Hinweises auf die Patenterteilung: **02.11.2005**
(21) Anmeldenummer: 03717027.1
(22) Anmeldetag: 30.04.2003
(51) Int. Cl.: A61K 35/78

(54) **VERFAHREN ZUR HERSTELLUNG VON TOCOTRIENOL-ANGEREICHERTEN PRÄPARATIONEN**
METHOD FOR PRODUCING PREPARATIONS RICH IN TOCOTRIENOL
PROCEDE DE PRODUCTION DE PREPARATIONS ENRICHIES EN TOCOTRIENOL

(30) Priorität: 03.05.2002 AT 6852002
(43) Veröffentlichungstag der Anmeldung: 02.02.2005
(73) Patentinhaber: vis-vitalis Lizenz- und Handels GmbH, 5020 Salzburg (AT)
(72) Erfinder: KÖSSLER, Peter, A-5571 Mariapfarr (AT); FUCHS, Norbert, A-5571 Mariapfarr (AT); SADEGHI, Behzad, A-1100 Wien (AT)
(74) Vertreter: Sonn & Partner Patentanwälte
(86) Internationale Anmeldenummer: PCT/AT2003/000125
(87) Internationale Veröffentlichungsnummer: WO 2003/092709

(56) Entgegenhaltungen:
- EP-A- 0 770 324
- WO-A-02/50054
- FRANZEN J; HAASS M.: "Vitamin E Content during Development of some Seedlings" PHYTOCHEMISTRY, Bd. 30, Nr. 9, 1991, Seiten 2911-2913, XP001154623
- ANDARWULAN N; FARDIAZ D; WATTIMENA G; SHETTY, K.: "Antioxidant Activity Associated with Lipid and Phenolic Mobilization during Seed Germination of Pangium edule Reinw." JOURNAL OF AGRICULTURAL AND FOOD CHEMISTRY, Bd. 47, Nr. 8, 1999, Seiten 3158-3163, XP002252812

## Beschreibung

Die Erfindung betrifft ein Verfahren zur Herstellung von Tocotrienol-angereicherten Präparationen.

Während sich die konventionelle Ernährungswissenschaft während der letzten Jahrzehnte darauf beschränkte, Nährstoffe (Proteine, Kohlenhydrate, Fette, Vitamine, Mineralstoffe und Spurenelemente) primär im Hinblick auf ihre "ernährenden" Eigenschaften (also auf die Gewährleistung des Bau- und Betriebsstoffwechsels) zu untersuchen, rückten während der letzten Jahre sogenannte sekundäre Pflanzenstoffe (phytochemicals) zunehmend in den Blickpunkt des ernährungswissenschaftlichen Interesses. Werden diese natürlichen Substanzen definitionsgemäß zwar nicht zu den klassischen Nährstoffen gerechnet, so üben sie dennoch zahlreiche biologische Wirkungen aus, weshalb sie auch als "bioaktive Pflanzenstoffe" bezeichnet werden.

Tocotrienole zählen zu dieser Kategorie von Pflanzenstoffen, obwohl sie auch zu den Vitaminen gerechnet werden, da sie - wenn auch geringe - Vitamin E-Aktivität aufweisen. Abgesehen von dieser Vitamin E-Aktivität weisen Tocotrienole cholesterinsenkende, zellschützende und antioxidative Eigenschaften auf. Antioxidantien sind durch ihre Fähigkeit, auf molekularer Ebene Elektronen an Partnermoleküle abzugeben, charakterisiert, wobei diese Fähigkeit zur Elektronenabgabe für definierte Einzel-Molekül-Verbindungen durch das sogenannte Standard-Redoxpotential quantifiziert wird, für Antioxidantien-Gemische dagegen durch die sogenannte antioxidative Kapazität (reduktive Kapazität) ausgedrückt wird. Das antioxidative Potential von Tocotrienolen wird im Vergleich zu synthetischen Tocopherolen - je nach Untersuchungsmedium und Untersuchungsmethode - als fünfzig- bis eintausendfach höher beschrieben.

Tocotrienole sind aufgrund ihres Molekülaufbaus ausschließlich fettlöslich, sodass sie Ihre biologische Aktivität im humanen und tierischen Gewebe vor allem an den lipophilen Kompartimenten (intra- und extrazelluläre Biomembranen) entfalten. Lipophile Antioxidantien haben daher eine wichtige biologische Rolle im Rahmen des antioxidativen Schutzes von Zellkernen (genetisches Material), der Mitochondrien (zelluläre Energieversorgung), des endoplasmatischen Retikulums (zelluläre Syntheseleistung) sowie an der Zellmembran (Stabilität und Lebensdauer von Geweben). Aus diesem Grunde kommt den Tocotrienolen für den Schutz des genetischen Materials (Schutz vor Mutationen durch schädigende Peroxide, Radikale und Xenobiotika), für die optimale zelluläre Ener gieversorgung (Leistungsfähigkeit von Immun- und Organzellen), für die zelluläre Syntheseleistung (Regenerationspotential des Immunsystems und von Geweben) sowie für die Funktionsfähigkeit und Lebensdauer aller Körperzellen eine enorme - über den basalen Ernährungszweck hinausgehende - Bedeutung zu. Insbesondere auch das Nervensystem, das Zentralnervensystem ebenso wie das periphere Nervensystem, welches zu mehr als 50 % aus lipoiden Substanzen besteht, ist auf einen ausreichenden und permanenten Schutz seiner Strukturen durch lipophile Antioxidantien angewiesen. Die ernährungsmedizinischen Anwendungsbereiche von Tocotrienolen umfas sen daher das Immunsystem (Allergien, Krebs) ebenso wie das cardiovaskuläre System (Angina pectoris, Herzinfarkt- Vor- und Nachsorge), den Muskel/Sehnen/Gelenks-Apparat (degenerative Muskel- und Gelenkserkrankungen), die Leber als Entgiftungsorgan (umweltbedingte Erkrankungen), die Haut (atopische Erkrankungen, Ageing) und letztlich regenerative Prozesse des Nervensystems (Multiple Sklerose, Morbus Alzheimer, Morbus Parkinson, spinale und peripher-neurologische Erkrankungen und Verletzungsfolgen).

Es besteht daher ein Bedarf an der Bereitstellung von Tocotrienol-hältigen Produkten, insbesondere an natürlichen Produkten mit besonders reichem Tocotrienol-Gehalt.

Die Erfindung betrifft daher ein Verfahren zur Herstellung von Tocotrienol-angereicherten Präparationen, welches die folgenden Schritte aufweist:
- Inkubieren von Pflanzensamen mit einer Elektrolyt-Nährlösung, so dass Tocotrienol-angereicherte Pflanzenkeimlinge gebildet werden,
- Gewinnen der Pflanzenkeimlinge und
- Extrahieren einer Tocotrienol-hältigen Präparation aus den gewonnenen Pflanzenkeimlingen, insbesondere aus der Kleie und dem Keim der gewonnenen Pflanzenkeimlinge.

Es zeigte sich, dass mit dem erfindungsgemäßen Verfahren der Tocotrienol-Gehalt von Pflanzenkeimlingen entscheidend erhöht werden kann, obgleich andere, nah verwandte antioxidative sekundäre Pflanzenstoffe, wie z.B. Tocopherole, während der Keimung, insbesondere während der erfindungsgemäß vorgenommenen Keimung, z.T. stark reduziert werden. Prinzipiell wird im Zuge der Keimung allgemein davon ausgegangen, dass sich zwar der Bedarf des keimenden Samens an Baumaterial und antioxidativen Schutzmolekülen für diesen Prozess erhöht, dass aber dieser erhöhte Bedarf entweder aus den vorhandenen oder unmittelbar neugebildeten Stoffen umgehend verbraucht wird. Umso überraschender war es, dass eine ganz bestimmte, besonders wertvolle Klasse von Verbindungen, die Tocotrienole, durch die Keimung in elektrolytangereicherter Nährlösung selektiv angereichert werden konnte.

Die Keimung von Pflanzensamen in Elektrolyt-Nährlösungen ist z.B. in den EP 0 770 324 A und EP 0 799 578 A beschrieben. Es war bekannt, dass die dabei erhaltenen Keimlinge zwar einen erhöhten Elektrolytgehalt aufweisen, dass jedoch der Verbrauch an antioxidativen Schutzmolekülen ebenfalls erhöht und deren Gehalt im Keimling daher reduziert ist.

Gemäß der US 5 908 940 A wird ein spezielles Verfahren zur Herstellung von "Tocol"-Produkten (Tocotrienol, Tocopherol und Tocotrienol-ähnliche Verbindungen) beschrieben, bei welchem pflanzliches Rohmaterial bei 80-150°C für 30 Minuten bis 4 Stunden trocken erhitzt wird, worauf die gewünschten Inhaltsstoffe extrahiert werden. Als pflanzliches Ausgangsmaterial ist dabei eine ganze Reihe von verschiedensten Materialien erwähnt. Dabei ist aber weder das Keimen des Ausgangsmaterials (zur Herstellung von Elektrolyt-angereicherten Keimlingen) noch das Anreichern des Ausgangsmaterials mit anorganischen Nährstoffen vorgesehen. Das dort beschriebene Verfahren zielt vielmehr darauf ab, durch die Hitzeeinwirkung pflanzliche Enzyme zu inaktivieren, um einen Abbau von Tocopherolen und Tocotrienolen zu verhindern. Der zentrale überraschende Aspekt der vorliegenden Erfindung, wonach gerade Tocotrienole in speziell Elektrolyt-angereicherten Keimlingen in erhöhter Ausbeute gewonnen werden können, jedoch beispielsweise der Tocopherol-Gehalt gleichzeitig erniedrigt ist, geht aus diesem Dokument weder hervor noch wird dies durch dieses Dokument nahegelegt.

Die EP 0 616 810 A1 betrifft die Verwendung von keimendem Reis als Arzneimittel, insbesondere zur Prophylaxe und Therapie von Krebs. In diesem Dokument wird jedoch weder allgemein auf einen Tocotrienol-Gehalt Bezug genommen wird, noch kann irgendein Hinweis herausgelesen werden, dass gerade in (Reis-)Keimlingen ein erhöhter Tocotrienol-Gehalt festzustellen ist. Weiters geht aus diesem Dokument die Keimung in Elektrolyt-angereicherten Medien nicht hervor.

Die Elektrolyt-Nährlösung enthält dabei vorzugsweise - unabhängig voneinander - 1 mg/l oder mehr, bevorzugter 10 mg/l oder mehr, insbesondere 50 mg/l oder mehr, Zink-, Eisen-, Kalium- und/oder Magnesiumionen, 0,5 mg/l oder mehr, bevorzugter 5 mg/l oder mehr, insbesondere 25 mg/l oder mehr, Kupfer-, Mangan-, Strontium- und/oder Lithiumionen, 0,1 mg/l oder mehr, bevorzugter 1 mg/l oder mehr, insbesondere 5 mg/l oder mehr, Selen-, Molybdän-, Chrom-, Arsen-, Vanadium- und/oder Kobaltionen.

Prinzipiell ist das erfindungsgemäße Verfahren auf viele verschiedene Arten von Pflanzensamen anwendbar, erfindungsgemäß bevorzugt sind aber selbstverständlich Samen, die entweder besonders hohe Tocotrienol-Gehalte ermöglichen, oder Samen von Pflanzen, die sich ganz besonders gut für großindustrielle Realisierung des erfindungsgemäßen Verfahrens eignen. Vorzugsweise werden daher die Pflanzensamen ausgewählt aus Walnuss-, Weizen-, Sonnenblumen-, Palmen-, Roggen-, Gersten-, Hafer-, Amaranth-, Quinoa-, Reissamen oder Mischungen dieser Samen.

Bevorzugter Weise werden die Pflanzenkeimlinge vor dem Extrahieren getrocknet. Dies erhöht nicht nur ihre Lagerfähigkeit, sondern macht die erfindungsgemäß erhaltenen Keimlinge für viele großtechnische Anwendungen geeignet.

Es ist weiters bevorzugt, wenn die Pflanzenkeimlinge vor dem Extrahieren gemahlen werden, da der Gehalt an wertbestimmenden Tocotrienolen und anderen lipophilen Antioxidantien sowie essen tiellen Fettsäuren in der Kleie und im Keim am höchsten ist. Dadurch können die Keimlinge besser in bewährte (Öl-)Extraktionsanlagen, insbesondere solche mit Druckseparatoren, überführt und extrahiert werden.

Aufgrund ihrer chemischen Struktur werden Tocotrienole-Präparate vorzugsweise als Öl gewonnen. Das Extrahieren wird daher bevorzugt unter Erhalt eines öligen Extrakts vorgenommen. Die Extraktion mittels organischer Lösungsmittel (selbst mit lebensmittel technologisch unbedenklichen organischen Lösungsmitteln) oder in (wässerigen) Suspensionen (z.B. mit Mizellen, etc.) ist zwar auch möglich, jedoch erfindungsgemäß nicht bevorzugt, da die erfindungsgemäßen Tocotrienol-Präparate vorzugsweise so natürlich, unverfälscht und biologisch wertvoll wie möglich zur Verfügung ge stellt werden sollen. Dazu ist es auch günstig, so viel wie möglich an den natürlichen Reaktionspartnern der Tocotrienole mitzuextrahieren, um bei der Anwendung am Menschen einen möglichst hohen biologischen Wirkungsgrad zu erzielen.

Besonders bevorzugt wird daher das erfindungsgemäße Extrahieren mit überkritischem CO₂ vorgenommen. Die fettlöslichen Bestandteile können aber auch z.B. mit Hexan oder anderen organischen Lösungsmitteln extrahiert werden.

Da bei zu langer Inkubation mit der Nährlösung der Gehalt der Keimlinge an Tocotrienolen wieder sinken kann, ist es erforderlich, dass die Inkubationsdauer mit der Nährlösung für jeden Samentyp optimiert wird. Dies ist jedoch für den Fachmann ohne weiteres möglich, z.B. durch die im folgenden Beispielabschnitt geoffenbarten Inkubations- und Analysenverfahren. Vorzugsweise wird die Inkubationsdauer so gewählt, dass damit ein optimaler Gehalt an Tocotrienolen erhalten werden kann. Vorzugsweise wird so lange inkubiert, dass der Gehalt an Tocotrienolen gegenüber dem Gehalt im ungekeimten Samen um zumindest 100%, insbesondere um zumindest 300% erhöht ist.

Das Inkubieren findet dabei bei Temperaturen und Bedingungen statt, die für die übliche Keimung von Samen der gewählten Art geeignet oder erprobt sind. Gemäß einer bevorzugten Ausführungsform wird das erfindungsgemäße Inkubieren bei einer Temperatur von 10 bis 40°C, vorzugsweise 15 bis 30°C, insbesondere 19 bis 21°C, vorgenommen.

Die Elektrolyt-Nährlösung, mit der die Pflanzensamen inkubiert werden, enthält vorzugsweise Vanadium-, Selenat-, Molybdat-, Cobalt-, Chrom-(III)-, Mangan-, Strontium-, Lithium-, Kupfer-, Eisen-(III)-, Zink-, Gluconat-, Citrat-, Lactat-Ionen oder Kombinationen dieser Ionen in einer Menge von 0,1 bis 1000 mg, vorzugsweise von 1 bis 500 mg, insbesondere von 3 bis 100 mg.

Das Extrahieren gemäß der vorliegenden Erfindung kann mit einer Vielzahl an geeigneten Vorrichtungen und Verfahren, jeweils angepasst an die gewählten Samen bzw. Keimlinge, erreicht werden. Besonders bewährt hat sich erfindungsgemäß die Extraktion mittels Autoklaven und Druckseparatoren. Diese können vorzugsweise, unabhängig voneinander, bei einem Autoklavendruck von 100 bar oder mehr, vorzugsweise von 200 bar oder mehr, insbesondere von 250 bar oder mehr, bei einem Separatordruck von 20 bar oder mehr, vorzugsweise 30 bar oder mehr, insbesondere 45 bar oder mehr, bei einer Autoklaventemperatur von 30°C oder mehr, vorzugsweise 40°C oder mehr, insbesondere 50°C oder mehr, und bei einer Separatortemperatur von 20°C oder mehr, vorzugsweise von 30°C oder mehr, insbesondere von 40°C oder mehr, betrieben werden.

Je nach Inkubationsdauer und Verbrauch der Nährlösung kann das Inkubieren in bevorzugten Fällen unter zumindest einmaligem, vorzugsweise zumindest zweimaligem, insbesondere zumindest dreimaligem Wechsel der Nährlösung durchgeführt werden.

Gemäß einem weiteren Aspekt betrifft die vorliegende Erfindung Tocotrienol-angereicherte Präparationen aus dem Keim und/oder der Kleie von Pflanzenkeimlingen, erhältlich nach dem erfindungsgemäßen Verfahren. Besonders bevorzugt sind dabei Tocotrienol-angereicherte Weizenkeimlings-Präparationen aus dem Keim und/oder der Kleie von Weizenkeimlingen, die einen Tocotrienol-Gehalt von 500 mg/kg Trockenmaterial oder mehr, vorzugsweise von 1000 mg/kg Trockenmaterial, insbesondere von 2000 mg/kg Trockenmaterial, aufweisen. Weiters sind auch Tocotrienol-angereicherte Gerstenkeimlings-Präparationen aus dem Keim und/oder der Kleie von Gerstenkeimlingen bevorzugt, die einen Tocotrienol-Gehalt von 1500 mg/kg Trockenmaterial oder mehr, vorzugsweise einen gamma-Tocotrienol-Gehalt von 500 mg/kg Trockenmaterial, insbesondere einen gamma-Tocotrienol-Gehalt von 200 mg/kg Trockenmaterial, aufweisen.

Aus den erfindungsgemäßen Tocotrienol-angereicherten Präparationen lassen sich Tocotrienol-hältige Präparate herstellen, die einen besonders wichtigen ernährungswissenschaftlichen Aspekt betreffen und insbesondere als biologisch wertvolles effektives Antioxidans wirken können, da darin der erhöhte Tocotrienol-Gehalt nicht nur als isolierte (erhöhte) Tocotrienol-Gabe wirkt, sondern dadurch, dass die Tocotrienole erfindungsgemäß mit ihren natürlichen Wirkungspartnern (insbesondere Redoxpartnern) verabreicht werden können, mit diesen Partnern auch ungleich effektiver wirken.

Je nach Bedarf kann das Tocotrienol-hältige Präparat gemäß der vorliegenden Erfindung bevorzugterweise zusätzlich mit pharmazeutischen Wirkstoffen, pharmazeutischen Hilfsstoffen, lebensmitteltechnischen Produkten oder lebensmitteltechnischen Zusatzstoffen versetzt werden. Dabei sollte aber vorzugsweise darauf Bedacht genommen werden, dass mit einer derartigen Zugabe das "natürliche Gleichgewicht" zwischen Tocotrienolen und den angesprochenen natürlichen Wirkungspartnern nicht wesentlich beeinträchtigt wird.

Die vorliegende Erfindung wird durch die nachfolgenden Beispiele, auf die sie jedoch nicht beschränkt sein soll, näher erläutert.

### Beispiel 1:

### Verfahren zur Gewinnung Tocotrienol-reicher Öle aus keimendem Getreide:

Bestimmte Pflanzenöle wie Walnussöl, Weizenkeimöl oder Sonnenblumenöl gelten als besonders reich an Tocopherolen, insbesondere an D,L-beta-Tocopherol. Hohe Gehalte an antioxidativen Tocotrienolen dagegen weisen Palmöl auf, ebenso Roggen, Gerste, Hafer, Weizenkleie und Reis. Bekanntlich erhöhen keimende Getreide- und Leguminosen-Samen auf endogenem Wege ihre Vitamin-Gehalte, um der gesteigerten Syntheseleistung während des Keimungsvorganges Rechnung zu tragen.

Daneben erhöhen sich im Zuge der gesteigerten Zell-Neubildung auch die mehrfach ungesättigten Fettsäuren um beinahe 50%, um für die Neubildung der Zellen biologisch wertvolles "Baumaterial" im ausreichenden Maße zur Verfügung stellen zu können. Mehrfach ungesättigte Fettsäuren sind jedoch sehr oxidationsempfindlich gegenüber Licht und Sauerstoff, sodass keimende Samen zum Schutz dieser biologisch wertvollen Fettsäuren auch entsprechende Mengen an lipophilen Antioxidantien (Tocotrienolen) synthetisieren. Ziel der vorliegenden Versuche war es, die Veränderung von Tocopherol- und Tocotrienol-Gehalten während des Keimungsvorganges qualitativ und quantitativ zu erfassen und nach Möglichkeiten zu suchen, insbesondere die Gehalte an antioxidativen Tocotrienolen während des Keimungsvorganges gezielt zu erhöhen.

Die nachfolgende Tabelle 1 zeigt die gaschromatographisch ermittelten Gehalte an (hoch) ungesättigten Fettsäuren des in Tabelle 5 angeführten "Gerstenkleieöles, 24 Stunden mit Nährlösung gekeimt":

**Tabelle 1**

| **Chemische Untersuchungen** | |
|---|---|
| **Art** | **Werte** |
| Wasser (grav.) % | 4,9 % |
| Säurezahl (titr.) | 8,8 |
| Freie Fettsäure ber. % | 4,4 % |
| Peroxidzahl nach Wheeler | 4,1 |

| **Zusammensetzung des Fettsäurenmethylestergemisches:** **(gaschromatographisch)** | |
|---|---|
| Palmitinsäure | 19,8 % |
| Stearinsäure | 1,1 % |
| Ölsäure | 19,5 % |
| Linolsäure | 53,6 % |
| Linolensäure | 4,1 % |
| Arachinsäure | 0,3 % |
| Behensäure | 0,3 % |
| Erucasäure | 0,1 % |
| Lignocerinsäure | 0,2 % |
| Eicosapentaensäure (C20 5=) | 0,2 % |
| Asche (grav.) | < 0,1 g/100 g |
| Fett (nach Weibull) | 94,6 g/100 g |
| Gesättigte Fettsäuren | 20,5 g/100 g |
| einfach unges. Fettsäuren | 19,3 g/100 g |
| mehrfach unges. Fettsäuren | 54,8 g/100 g |
| Petroletherunlösliches | 1,0 % |
| Seifengehalt | 0,3 % |

### Keimversuche:

Keimfähige Weizen- und Gersten-Samen wurden alternativ mit destilliertem Wasser oder mit einer Nährlösung über einen Zeitraum von 24 bzw. 96 Stunden gekeimt.

Die Nährlösung enthielt folgende gelöste Nährsalze (Angaben im mg/l):

**Tabelle 2**

| | |
|---|---|
| Vanadiumoxidsulfat 5 H₂O | 24,85 |
| Natriumselenat | 11,95 |
| Natriummolybdat | 12,60 |
| Cobaltchlorid 6 H₂O | 20,20 |
| Chrom-III-chlorid | 25,60 |
| Manganchlorid | 73,75 |
| Strontiumlactat | 84,25 |
| Lithiumchlorid | 152,75 |
| Kupfergluconat | 178,50 |
| Ammonium-Eisen-III-citrat | 178,50 |
| Zinkgluconat | 394 |

Vor der eigentlichen Keimphase wurden die Getreidesamen zwölf Stunden in den entsprechenden Lösungen eingeweicht. Die Keimung erfolgte bei Raumtemperatur (19-21°C) und normalen Tag/Nacht-Lichtverhältnissen in handelsüblichen Keimgeräten, welche aus durchsichtigen, übereinander angeordneten Plastikschalen mit Ablaufvorrichtung bestanden. Während der Keimung wurden die Keimlinge zweimal täglich mit den entsprechenden Lösungen (also de stilliertem Wasser bzw. Nährlösung, jeweils 250 ml/90 g) gespült. Nach der Ernte wurden die Keimlinge gründlich mit zweifach destilliertem Wasser gespült (dreimal mit ca. 800 ml) und anschließend bei 60°C unter Heißluft getrocknet. Nach dem Trocknungsvorgang wurden die gekeimten Samen gemahlen und die daraus erhaltenen Kleien mittels überkritischem CO₂ extrahiert.

Die Extraktionsparameter zur Gewinnung der öligen Fraktion aus den Getreidekeimen und -kleien waren:

Für die in nachstehender Tabelle 3 angeführten Untersuchungsproben 1 - 4:

**Tabelle 3**

| | |
|---|---|
| Extraktionszeit | > 210 Minuten |
| Autoklavendruck | > 280 bar |
| Druckseparator 1 | 65 bar |
| Druckseparator 2 | 42 bar |
| Autoklaventemperatur | 65°C |
| Temperaturseparator 1 | 43°C |
| Temperaturseparator 2 | 26,8°C |

Die Ölgehalte der Kleien betrugen 2,2 bis 3,6 Gewichtsprozent.

Für die in nachstehender Tabelle 4 angeführten Untersuchungsproben 5 - 7:

**Tabelle 4**

| Einstufige Extraktion mit einstufiger Extraktabscheidung | |
|---|---|
| Autoklavendruck | 260 bar |
| Druckseperator | 50 bar |
| Autoklaventemperatur | 45-50°C |
| Temperaturseperator | 35°C |

Gesamt-CO₂ und CO₂-Fluss sind von der jeweils eingesetzten Rohware abhängig.

### Beispiel 2:

### Bestimmung der Tocopherole und Tocotrienole in den Keim- und Kleie-Ölen:

Die Tocopherole und Tocotrienole wurden nach Verseifung des Probenmaterials und nach Extraktion in n-Hexan mittels HPLC getrennt und anhand der Retentionszeiten mit einem Fluoreszenz-Detektor nachgewiesen. Die quantitative Auswertung erfolgte durch einen Vergleich der Peak-Flächen nach der externen Standardmethode. Als stationäre Phase diente eine HPLC-Säule 250x4.6MMX1/4"VALCO; LiChrosorb Si60-5, als mobile Phase diente eine Mischung von n-Hexan und Dioxan (95:5), als Vergleichs-Standards wurden Tocopherol und Tocotrienol der Firma Calbiochem verwendet.

**Tabelle 5**

| | | | | | | | | | | | |
|---|---|---|---|---|---|---|---|---|---|---|---|
| Die Vergleichsanalysen der einzelnen Proben ergaben (alle Werte in mg/kg Untersuchungsprobe): | | | | | | | | | | | |

| **Untersuchungsprobe** | α-T¹⁾ | β-T²⁾ | γ-T³⁾ | δ-T⁴⁾ | α-T3⁵⁾ | β-T3⁶⁾ | γ-T3⁷⁾ | δ-T3⁸⁾ | Σ T⁹⁾ | Σ T3¹⁰⁾ | Σ E¹¹⁾ |
|---|---|---|---|---|---|---|---|---|---|---|---|
| 1. Weizenkeimöl aus ungekeimtem Kom, extrahiert mit Druckse-parator 2 | 3276 | 0 | 1048 | 0 | 0 | 124 | 0 | 0 | 4324 | 124 | 4448 |
| 2. Weizenkeimöl, 96 Stunden mit Nährlösung gekeimt, extrahiert mit Druckseparator 2 | 1339 | 283 | 14 | 14 | 833 | 1075 | 167 | 34 | 1650 | 2190 | 3759 |
| 3. Weizenkteieöt aus ungekeimtem Kom, extrahiert mit Druckse-parator 1 | 630 | 222 | 3 | 4 | 358 | 804 | 52 | 11 | 859 | 1224 | 2083 |
| 4. Weizenkleleöl, 96 Stunden mit Nährlösung gekeimt, extrahiert mit Druckseparator 1 | 91 | 0 | 6 | 0 | 799 | 424 | 71 | 20 | 97 | 1314 | 1411 |
| 5. Gerstenkleieöl, 24 Stunden mit Wasser gekeimt | 264 | 0 | 149 | 12 | 893 | 140 | 275 | 33 | 425 | 1341 | 1766 |
| 6. Gerstenkleleöl, 24 Stunden mit Nährlösung gekeimt | 143 | 10 | 43 | 8 | 1028 | 251 | 559 | 60 | 204 | 1898 | 2102 |
| 7. Gerstenkleleöl, 96 Stunden mit Nährlösung gekeimt | 88 | 0 | 59 | 6 | 320 | 78 | 156 | 22 | 153 | 576 | 729 |

| | | | | | | | | | | | |
|---|---|---|---|---|---|---|---|---|---|---|---|
| Fußnoten: ¹⁾ alpha-Tocopherol, | | | | | | | | | | | |
| ²⁾ beta-Tocopherol, | | | | | | | | | | | |
| ³⁾ gamma-Tocopherol, | | | | | | | | | | | |
| ⁴⁾ delta-Tocopherol; | | | | | | | | | | | |
| ⁵⁾ alpha-Tocotrienol, | | | | | | | | | | | |
| ⁶⁾ beta-Tocotrienol, | | | | | | | | | | | |
| ⁷⁾ gamma-Tocotrienol, | | | | | | | | | | | |
| ⁸⁾ delta-Tocotrienol; | | | | | | | | | | | |
| ⁹⁾ Summe Tocopherol, | | | | | | | | | | | |
| ¹⁰⁾ Summe Tocotrienol, | | | | | | | | | | | |
| ¹¹⁾ Summe Vitamin E. | | | | | | | | | | | |

Die Analysenergebnisse zeigen:
- Einen Abfall der Tocopherole zugunsten eines Anstiegs der Tocotrienole während der Keimung, was auf eine Verminderung der zellulären Vitamin E-Aktivität bei gleichzeitigem Anstieg der antioxidativen Bedürfnisse während des Keimungsprozesses hinweist. Auf zellulärer Ebene bedeutet "Keimung" eine gesteigerte Aktivität auf enzymatischem Niveau. Die Pflanzenzelle benötigt ein Mehrfaches an biologisch hochwertigem Baumaterial, nämlich hochungesättigte Fettsäuren sowie Phospholipide für die Neubildung von Zellmaterial, also für das Wachstum. Aufgrund der Temperatur- und Sauerstoff-Empfindlichkeit dieses hochwertigen bio logischen Materials erhöht sich auch der Bedarf an antioxidativen Schutz-Molekülen. Die vorliegenden Analysenergebnisse zeigen, dass die pflanzliche Zelle diesem erhöhten Schutzbedürfnis durch Umwandlung von Tocopherolen zu Tocotrienolen Rechnung trägt.
- Eine Stimulierung der endogenen Tocotrienol-Synthese im keimenden Samen durch Zufuhr essentieller mineralischer Spurenelemente vor und während der Keimung: Während die Pflanzenzelle in der Lage ist - im Gegensatz zur humanen Zelle - ihren (gesteigerten) Bedarf an organischen Vitalstoffen (Vitaminen, Antioxidantien, hochungesättigten Fettsäuren) durch eigene Syntheseleistung selbstständig zu decken, ist sie - ebenso wie der Mensch - auf die exogene Zufuhr mineralischer Stoffe (Mineralstoffe, Spurenelemente) angewiesen. Wie die vorliegenden Untersuchungen zeigen, kann durch die Zufuhr mineralischer Spurenelemente der Tocotrienol-Gehalt von keimenden Samen im Vergleich zu herkömmlichen Keimungs-Methoden (also Keimung mit Wasser) signifikant erhöht werden.
- Eine Abnahme der Tocopherol- und Tocotrienol-Gehalte mit zunehmender Keimungsdauer am Beispiel der Gerstenkleie.

### Beispiel 3:

### Analyse der biologischen Qualität natürlicher Tocotrienol-Gemische:

Im Rahmen einer ex vivo-in vitro-Untersuchung wird die antioxidative Kapazität tocotrienolreichen Weizenkleie-Öls im Vergleich zu Weizenkeimöl, Tocopherolacetat und D-alpha-Tocopherol untersucht. Bei dieser Untersuchungsmethode wird humanes Serum der oxidativen Belastung einer definierten Menge von para-Benzochinon ausgesetzt. Durch Zugabe definierter Mengen der zu untersuchenden Antioxidantien (natürliches Tocotrienol-Gemisch aus Weizenkleie-Öl, Weizenkeimöl, Tocopherolacetat, D-alpha-Tocopherol) wird die antioxidative Belastbarkeit der Untersuchungsprobe durch kolorimetrische Bestimmung des aus para-Benzochinon reduzierten para-Dihydrochinon quantitativ ermittelt. Nach dieser Untersuchung wies das Tocotrienol-Gemisch aus gekeimter Weizenkleie eine um den Faktor 500 stärkere antioxidative Kapazität im Vergleich zu Tocopherolacetat und eine um den Faktor 1000 stärkere antioxidative Kapazität als D-alpha-Tocopherol auf.

### Diese Versuche wurden wir folgt durchgeführt:

Humanes Spender-Blut wird venös ohne Zusätze gewonnen und nach 1 Stunde Abstehen im Kühlschrank (ca. +4°C - +7°C) bei 800 - 1000 g zentrifugiert (Dauer 10 Minuten). Die separierten Serum-Fraktionen werden abgenommen und gepoolt. Das Serum kann bei -22°C für ca. 14 Tage gelagert oder sofort für die erforderlichen Messungen eingesetzt werden.

Zur Bestimmung der antioxidativen Kapazität eines Antioxidans wird das Serum in Stufen radikalisch belastet. Zur Radikalbildung wurde p-Benzochinon eingesetzt.

Im physiologischen Milieu (pH = leicht alkalisch) wandelt sich diese Substanz in relativ stabile Radikal-Anion des Chinhydron-Systems um. Dabei wird ein Wasserstoffatom (1 Proton/1 Elektron) von p-Benzochinon aufgenommen.

Die weitere Reaktion zum stabilen Endprodukt p-Dihydrochinon erfolgt bei pH 6,9 - 7,4 in Minuten und primär linear dem Antioxidantiengehalt (= Reduktionsmittel) im Reaktionsmilieu umgekehrt proportional.

Somit sind zur Bestimmung des Endproduktes (Dihydrochinon) als typisch gefärbte Substanz die Bedingungen der Gültigkeit des Lambert-Beer'schen Gesetzes erfüllt.

Das relativ stabile Radikal-Anion (Chinhydron) wird unter Aufnahme eines weiteren Elektrons (Wasserstoff der verfügbaren Antioxidantien) in einem zweiten Reduktionsschritt in das Dihydrochinon überführt. Die Reaktion ist kolorimetrisch verfolgbar, da der Übergang in die völlig reduzierte Substanz (Dihydrochinon) mit einer starken Farbvertiefung verbunden ist.

Die kolorimetrische Bestimmung der Menge an Endprodukt erfolgt bei λ = 500 nm. Die Extinktion bei dieser Wellenlänge ist dann der Menge an Reaktions-Endprodukt bzw. auch der Menge an umgesetztem Antioxidans zur Umwandlung der radikalischen Zwischenstufe direkt proportional.

Aus den stöchiometrischen Reaktionsverhältnissen lassen sich bei linearer Extinktionszunahme und linear wachsendem Verbrauch des antioxidativen Wirkstoffs die umgesetzen Anteile genau berechnen.

Nach Michaelis/Kalkar bzw. Pauling (Holleman-Wiberg; Lehrbuch der Anorganischen Chemie, de Gruyter-Verlag (1995)) hängt die Reaktionsintensität bis zum Dihydrochinon vom-Verhältnis der Konzentrationen oxidiert zu reduziert ab. D.h.: je höher das Reduktions-(Antioxidantien-)Angebot ist, um so stärker wird die Reaktion gehemmt, der Gehalt an entstehendem Dihydrochinon nimmt ab. Damit ist die Anwendung dieser Reaktion zur Bestimmung feindosierter quantitativer Aussagen für reduzierend wirkende Substanzen in Redoxsystemen hervorragend geeignet.

### Vorgehensweise:

In drei parallelen Meßserien werden Serumproben (je 500 ml) des Pools mit p-Benzochinon in drei Stufen (10, 20 und 30 µg) belastet. Nach 30, 60, 90 und 180 Sekunden Reaktionszeit werden jeweils die Extinktionswerte bei λ = 500 nm ermittelt. Der Mittel wert des 60 Sekunden-Wertes entspricht bei den errechneten Umsetzungsquantitäten dem definierten Titer (= Eindrittel-Umsatz). Dieser Wert ergibt sich präzise aus der graphischen Darstellung der Mittelwerte als Extinktionskurve. Auf diesen bestimmten Eichwert des Serumpools ohne Antioxidantienzusatz werden alle weiteren Untersuchungen mit Antioxidantien-Zugabe vergleichend bezogen (mit ansteigendem Angebot an Antioxidantien im Serum muss die Extinktion für Dihydrochinon also abfallen).

Zur Feststellung der antioxidativen Kapazität des erfindungsgemäßen tocotrienolreichen Weizenkleie-Öls wurden drei parallele Meßserien an Serumproben unter Zusatz von 500 µg/ml erfindungsgemäßes Präparat durchgeführt. Hier ergab sich ein Abfall des Extinktionswertes (λ = 500 nm/60 sec) gegenüber dem unbehandelten Serum um im Mittel 0,017 Einheiten.

Unter Bezugnahme auf die Extinktionswertekurve des Vergleichs-Serums ohne Zusätze und die angewendeten stöchiometrischen Reaktionsverhältnisse (Serumbestimmung/Konzentration an p-Benzochinon/Menge pro ml Serum-Verdünnung) ergibt sich aus diesem Eich system der der Umrechnungsfaktor Extinktion zu radikalischer Hemmung.

Bei der angewendeten 1/50-Verdünnung der Serum-Vergleichsproben und der Eindrittelumsetzung (Extinktionsvergleich) der eingesetzten Molekül-Anzahl (zu berechnen aus der p-Benzochinonkonzentration pro Volumeneinheit nach der Loschmidt'schen Zahl) ergibt sich der Umrechnungsfaktor auf Chinhydron-Radikale. 0,017 Extinktionseinheiten entsprechen danach 0,017•10•28,08 = 4,914 µg Radikale pro ml/sec.

Bei dem Ansatz:
3 mg erfindungsgemäßes Präparat in 6 ml Serum, davon 0,5 ml verdünnt 1 : 10 isotonisch ergeben sich 250 µg in 5 ml. Die Extinktionsmessungen wurden in 1,5 ml davon unter Zugabe von 10 bzw. 20 und 30 µg p-Benzochinon durchgeführt, was einer Menge von 75 µg erfindungsgemäßem Präparat entspricht.
75 µg erfindungsgemäßes Präparat = 4,914 µg Radikale pro ml/sec. 1 mg erfindungsgemäßes Präparat = 65,52 µg Radikale sowie
1 g erfindungsgemäßes Präparat = 65,52 mg Radikale je ml und sec.
Als Entgiftungsleistung des erfindungsgemäßen Präparates.

Diese Aktivität kann beliebig mittels der Loschmidt'schen Zahl auf die Sauerstoff-Stufen umgerechnet werden:
1 g erfindungsgemäßes Öl = der Entgiftung von 23,28•10²⁰ OH^{.}-Radikalen (Atomgewicht = 17)
oder
12,37•10²⁰
O₂^{.} -Radikalen (Atomgewicht = 32)

Ein weiteres Kriterium zur wertenden Aussage über die antioxidative (Schutz-)Wirkung stellt (den Einfluss auf) die Redox-Pufferung dar. Physiologisch aktive Substanzen mit antioxidativer Wirkung stabilisieren oder steigern trotz zunehmender radikalischer Belastung die oxidative Belastbarkeit (Beschwerung) biologischer oxidoreduktiver Systeme (z.B. Serum).

Zur Bestimmung der Aktivität des erfindungsgemäßen Präparates zur Stabilisierung der oxidativen Belastbarkeit biologischer Systeme wurden wiederum je drei Testserien untersucht und daraus die Mittelwerte bestimmt:

### 1. Humanes vollserum (vital) ohne erfindungsgemäßem Präparat (Leerserum)

Serumproben von je 0,5 ml Volumen werden mit 10, 20 bzw. 30 µg p-Benzochinon belastet. Nach 30 Minuten Inkubation (20°C) werden die Redox-Potentiale (mV) gemessen, mit dem Leerwert-Potential (ohne p-Benzochinon) verglichen und als Pufferkurve graphisch dargestellt. Die Pufferfunktion ergibt sich als Linearfunktion zwischen den Potential-Punkten für 10, 20 und 30 µg Belastung durch p-Benzochinon.

### 2. Humanes Vollserum (vital) mit 500 µg erfindungsgemäßem Präparat pro ml

Die Serumproben zu je 0,5 ml werden ebenfalls mit 10 bzw. 20 und 30 µg p-Benzochinon belastet. Nach 30 Minuten Inkubation werden die Redox-Potentiale gemessen und, mit dem Potentialwert ohne p-Benzochinon verglichen, graphisch dargestellt. Auch hier ergibt sich die Pufferfunktion als lineare Gleichung der Verbindung der Punkte für 10, 20 und 30 µg p-Benzochinon.

### Ergebnis:

Für Serum mit Zusatz an erfindungsgemäßem Präparat ergibt sich ein Steigerungsverhältnis der Pufferkurve von +0,35 mV. Ohne erfindungsgemäßem Präparat beträgt das Steigerungsverhältnis der linearen Puffer-Funktion lediglich -0,35 mV.

Das erfindungsgemäße Produkt stellt somit ein hochgradig wirksames Antioxidans auch unter physiologischen Bedingungen (auch in vivo) dar.

Für eine erweiterte Bewertung der Produktqualität ist der Vergleich mit anderen Antioxidantien erforderlich. Zu diesem Zweck wurden wie oben beschrieben analog je drei Analysenserien für die Produkte:
Weizenkeimöl (kaltgepresst, 100 % reinste Apothekenqualität), D,L-alpha-Tocopherolacetat (synthetisch, 50 %) - (vis-vitalis (AT)),
alpha-Tocopherol (natürlich, 50 %) - (vis-vitalis (AT)), durchgeführt.

Die Ergebnisse werden in der nachfolgenden Tabelle 6 zusammengefasst:

**Tabelle 6**

| | Erfindungsgemäßes Weizenkeimöl | Weizenkeimöl | Tocopherolacetat | D-alpha-Tocopherol |
|---|---|---|---|---|
| Antiox-Titer | 0,19 | 0,14 | 0,125 | 0,06 |
| Radikale µg/ml·sec | 5,335 | 3,92 | 3,51 | 1,6848 |
| Tocotrienole bei 500 mg Substanz/ml | 850 µg | 1,4 mg | | 250 mg |
| 1 mg Tocotrienol - Wirkung in µg Radikale/ml·sec. | 6,276 | 2,8 | 0,014 | 0,00674 |
| Faktor der Wirksamkeit | 1 | 0,446 (1/2,24) | 0,0022 (1/450) | 0,001 (1/930) |

Aus allen Untersuchungen ergibt sich die folgende Feststellung:

Das erfindungsgemäße Präparat ist als antioxidatives Schutzprodukt effektiv wirksam und entfaltet ebenfalls eine hohe Schutzfunktion gegen Überoxidation auch unter physiologischen Bedingungen durch die Steigerung der Redoxpufferung (Beschwerung) biologischer Redox-Systeme.

### Schlussfolgerung:

Tocotrienole weisen im Gegensatz zu Tocopherolen zwar geringere Vitamin E-Aktivität auf, zeigen aber deutlich erhöhte antioxidative Leistungen. Die antioxidative Kapazität lipophiler Antioxidantien ist ein wichtiger Qualitätsparameter im ernährungsmedizinischen Einsatz bei Immun-, Herz/Kreislauf-, Muskel/Gelenks-, Leber-, Haut- und Nervenerkrankungen. Keime und Kleien von Getreide und Leguminosen-Samen weisen im Vergleich zu ungekeimten Samen geringere Tocopherol-, jedoch höhere Tocotrienol-Gehalte auf. Im Vergleich zu destilliertem Wasser lässt sich durch dieerfindungsgemäße Zugabe essentieller mineralischer Spurenelemente die pflanzliche Synthese von Tocotrienolen während des Keimungsvorganges deutlich stimulieren.

## Patentansprüche

1. Verfahren zur Herstellung von Tocotrienol-angereicherten Präparationen, **gekennzeichnet durch** die folgenden Schritte:
- Inkubieren von Pflanzensamen mit einer Elektrolyt-Nährlösung, so dass Tocotrienol-angereicherte Pflanzenkeimlinge gebildet werden,
- Gewinnen der Pflanzenkeimlinge und
- Extrahieren einer Tocotrienol-hältigen Präparation aus den gewonnenen Pflanzenkeimlingen, insbesondere aus der Kleie und dem Keim der gewonnenen Pflanzenkeimlinge.

2. Verfahren nach Anspruch 1, **dadurch gekennzeichnet, dass** die Pflanzensamen ausgewählt sind aus Walnuss-, Weizen-, Sonnenblumen-, Palmen-, Roggen-, Gersten-, Hafer-, Amaranth-, Quinoa-, Reissamen oder Mischungen dieser Samen.

3. Verfahren nach Anspruch 1 oder 2, **dadurch gekennzeichnet, dass** die Pflanzenkeimlinge vor dem Extrahieren getrocknet werden.

4. Verfahren nach einem der Ansprüche 1 bis 3, **dadurch gekennzeichnet, dass** die Pflanzenkeimlinge vor dem Extrahieren gemahlen werden.

5. Verfahren nach einem der Ansprüche 1 bis 4, **dadurch gekennzeichnet, dass** das Extrahieren unter Erhalt eines öligen Extrakts vorgenommen wird.

6. Verfahren nach einem der Ansprüche 1 bis 5, **dadurch gekennzeichnet, dass** das Extrahieren mit überkritischem CO₂ oder mit Hexan vorgenommen wird.

7. Verfahren nach einem der Ansprüche 1 bis 6, **dadurch gekennzeichnet, dass** das Inkubieren bei einer Temperatur von 10 bis 40°C, vorzugsweise 15 bis 30°C, insbesondere 19 bis 21°C, vorgenommen wird.

8. Verfahren nach einem der Ansprüche 1 bis 7, **dadurch gekennzeichnet, dass** ein Liter Nährlösung Vanadium-, Selenat-, Molybdat-, Cobalt-, Chrom-(III)-, Mangan-, Strontium-, Lithium-, Kupfer-, Eisen-(III)-, Zink-, Gluconat-, Citrat-, Lactat-Ionen oder Kombinationen dieser Ionen in einer Menge von 0,1 bis 1000 mg, vorzugsweise von 1 bis 500 mg, insbesondere von 3 bis 100 mg, enthält.

9. Verfahren nach einem der Ansprüche 1 bis 8, **dadurch gekennzeichnet, dass** das Extrahieren mittels Autoklaven und Druckseparatoren und, unabhängig voneinander, bei einem Autoklavendruck von 100 bar oder mehr, vorzugsweise von 200 bar oder mehr, insbesondere von 250 bar oder mehr, bei einem Separatordruck von 20 bar oder mehr, vorzugsweise 30 bar oder mehr, insbesondere 45 bar oder mehr, bei einer Autoklaventemperatur von 30°C oder mehr, vorzugsweise 40°C oder mehr, insbesondere 50°C oder mehr, und bei einer Separatortemperatur von 20°C oder mehr, vorzugsweise von 30°C oder mehr, insbesondere von 40°C oder mehr, vorgenommen wird.

10. Verfahren nach einem der Ansprüche 1 bis 9, **dadurch gekennzeichnet, dass** das Inkubieren unter zumindest einmaligem, vorzugsweise zumindest zweimaligem, insbesondere zumindest dreimaligem Wechsel der Nährlösung durchgeführt wird.

11. Tocotrienol-angereicherte Präparationen aus dem Keim und/oder der Kleie von Pflanzenkeimlingen, erhältlich nach einem Verfahren nach einem der Ansprüche 1 bis 10.

12. Tocotrienol-angereicherte Weizenkeimlings-Präparationen aus dem Keim und/oder der Kleie von Weizenkeimlingen, **dadurch gekennzeichnet, dass** sie einen Tocotrienol-Gehalt von 500 mg/kg Trockenmaterial oder mehr, vorzugsweise von 1000 mg/kg Trockenmaterial, insbesondere von 2000 mg/kg Trockenmaterial, aufweisen.

13. Tocotrienol-angereicherte Gerstenkeimlings-Präparationen aus dem Keim und/oder der Kleie von Gerstenkeimlingen, **dadurch gekennzeichnet, dass** sie einen Tocotrienol-Gehalt von 1500 mg/kg Trockenmaterial oder mehr, vorzugsweise einen gamma-Tocotrienol-Gehalt von 500 mg/kg Trockenmaterial, insbesondere einen beta-Tocotrienol-Gehalt von 200 mg/kg Trockenmaterial, aufweisen.

14. Tocotrienol-hältiges Präparat, enthaltend Tocotrienol-angereicherte Präparationen nach einem der Ansprüche 11 bis 14.

15. Tocotrienol-hältiges Präparat nach Anspruch 14, **dadurch gekennzeichnet, dass** es zusätzlich pharmazeutische Wirkstoffe, pharmazeutische Hilfsstoffe, lebensmitteltechnische Produkte oder lebensmitteltechnische Zusatzstoffe aufweist.

## Claims

1. A method of producing tocotrienol-enriched preparations,
**characterised by** the following steps:
- incubating plant seeds with an electrolyte nutrient solution so as to form tocotrienol-enriched plant embryos,
- recovering the plant embryos, and
- extracting a tocotrienol-containing preparation from the recovered plant embryos, in particular from the bran and from the germs of the recovered plant embryos.

2. A method according to claim 1, **characterised in that** the plant seeds are selected from walnut, wheat, sunflower, palm, rye, barley, oat, amaranth, quinoa, rice seeds or mixtures of these seeds.

3. A method according to claim 1 or 2, **characterised in that** the plant embryos are dried prior to extraction.

4. A method according to any one of claims 1 to 3, **characterised in that** the plant embryos are ground prior to extraction.

5. A method according to any one of claims 1 to 4, **characterised in that** the extraction is carried out by obtaining an oily extract.

6. A method according to any one of claims 1 to 5, **characterised in that** the extraction is effected with super-critical CO₂ or with hexane.

7. A method according to any one of claims 1 to 6, **characterised in that** incubation is carried out at a temperature of from 10 to 40°C, preferably 15 to 30°C, in particular 19 to 21°C.

8. A method according to any one of claims 1 to 7, **characterised in that** one litre of nutrient solution contains vanadium, selenate, molybdate, cobalt, chromium(III), manganese, strontium, lithium, copper, iron(III), zinc, gluconate, citrate, lactate ions, or combinations of these ions, in an amount of from 0.1 to 1000 mg, preferably from 1 to 500 mg, in particular from 3 to 100 mg.

9. A method according to any one of claims 1 to 8, **characterised in that** the extraction is carried out by means of autoclaves and pressure separators and, independently of each other, at an autoclave pressure of 100 bar or more, preferably 200 bar or more, in particular 250 bar or more, at a separator pressure of 20 bar or more, preferably 30 bar or more, in particular 45 bar or more, at an autoclave temperature of 30°C or more, preferably 40°C or more, in particular 50°C or more, and at a separator temperature of 20°C or more, preferably 30°C or more, in particular 40°C or more.

10. A method according to any one of claims 1 to 9, **characterised in that** incubating is carried out with at least once, preferably at least twice, in particular at least three times changing the nutrient solution.

11. Tocotrienol-enriched preparations from the germ and/or from the bran of plant embryos, obtainable by a method according to any one of claims 1 to 10.

12. Tocotrienol-enriched wheat embryo preparations from the germ and/or from the bran of wheat embryos, **characterised in that** they have a tocotrienol content of 500 mg/kg dry material or more, preferably 1000 mg/kg dry material, in particular 2000 mg/kg dry material.

13. Tocotrienol-enriched barley embryo preparations from the germ and/or from the bran of barley embryos, **characterised in that** they have a tocotrienol content of 1500 mg/kg dry material or more, preferably a gamma-tocotrienol content of 500 mg/kg dry material, in particular a beta-tocotrienol content of 200 mg/kg dry material.

14. A tocotrienol-containing preparation, comprising tocotrienol-enriched preparations according to any one of claims 11 to 14.

15. A tocotrienol-containing preparation according to claim 14, **characterised in that** it additionally comprises pharmaceutically active substances, pharmaceutical adjuvants, food-technological products or food-technological additives.

## Revendications

1. Procédé de fabrication de préparations enrichies au tocotriénol, **caractérisé par** les étapes suivantes :
- incubation de semences végétales avec une solution nutritive d'électrolyte de manière à former des plantules enrichies en tocotriénol,
- production des plantules et
- extraction d'une préparation contenant du tocotriénol des plantules produites, en particulier du son et du germe des plantules produites.

2. Procédé selon la revendication 1, **caractérisé en ce qu'**on choisit les semences végétales parmi les semences de noix, de blé, de tournesol, de palme, de seigle, d'orge, d'avoine, d'amarante, de quinoa, de riz ou les mélanges de ces semences.

3. Procédé selon la revendication 1 ou 2, **caractérisé en ce que** les plantules sont séchées avant l'extraction.

4. Procédé selon l'une quelconque des revendications 1 à 3, **caractérisé en ce que** les plantules sont broyées avant l'extraction.

5. Procédé selon l'une quelconque des revendications 1 à 4, **caractérisé en ce que** l'extraction est effectuée afin d'obtenir un extrait huileux.

6. Procédé selon l'une quelconque des revendications 1 à 5, **caractérisé en ce que** l'extraction est effectuée avec du CO₂ surcritique ou avec de l'hexane.

7. Procédé selon l'une quelconque des revendications 1 à 6, **caractérisé en ce que** l'incubation s'effectue à une température de 10 à 40°C, de préférence de 15 à 30°C, en particulier de 19 à 21°C.

8. Procédé selon l'une quelconque des revendications 1 à 7, **caractérisé en ce qu'**un litre de solution nutritive contient des ions vanadium, sélénate, molybdate, cobalt, chrome(III), manganèse, strontium, lithium, cuivre, fer(III), zinc, gluconate, citrate, lactate ou des combinaisons de ces ions en quantité de 0,1 à 1000 mg, de préférence de 1 à 500 mg, en particulier de 3 à 100 mg.

9. Procédé selon l'une quelconque des revendications 1 à 8, **caractérisé en ce qu'**on effectue l'extraction au moyen d'autoclaves et de séparateurs à pression et, indépendamment les uns des autres, à une pression en autoclave de 100 bars ou plus, de préférence de 200 bars ou plus, en particulier de 250 bars ou plus, dans une pression de séparateur de 20 bars ou plus, de préférence de 30 bars ou plus, en particulier de 45 bars ou plus, à une température d'autoclave de 30°C ou plus, de préférence de 40°C ou plus, en particulier de 50°C ou plus et à une température de séparateur de 20°C ou plus, de préférence de 30°C ou plus, en particulier de 40°C ou plus.

10. Procédé selon l'une quelconque des revendications 1 à 9, **caractérisé en ce que** l'incubation est réalisée en changeant au moins à une reprise, de préférence au moins à deux reprises, en particulier au moins à trois reprises la solution nutritive.

11. Préparations enrichies en tocotriénol à partir du germe et/ou du son de plantules, que l'on peut obtenir par un procédé selon l'une quelconque des revendications 1 à 10.

12. Préparations de plantules de blé enrichies en tocotriénol à partir du germe et/ou du son de plantules de blé, **caractérisées en ce qu'**elles présentent une teneur en tocotriénol de 500 mg/kg de matière sèche ou plus, de préférence de 1000 mg/kg de matière sèche, en particulier de 2000 mg/kg de matière sèche.

13. Préparations de plantules d'orge enrichies en tocotriénol à partir du germe et/ou du son de plantules d'orge, **caractérisées en ce qu'**elles présentent une teneur en tocotriénol de 1500 mg/kg de matière sèche ou plus, de préférence une teneur en gamma-tocotriénol de 500 mg/kg de matière sèche, en particulier une teneur en bêta-tocotriénol de 200 mg/kg de matière sèche.

14. Préparation contenant du tocotriénol, contenant des préparations enrichies en tocotriénol selon l'une quelconque des revendications 11 à 14.

15. Préparation contenant du tocotriénol selon la revendication 14, **caractérisée en ce qu'**elle présente, en plus des substances actives pharmaceutiques, des adjuvants pharmaceutiques, des produits agroalimentaires ou des additifs agroalimentaires.
